# EUROPÄISCHE PATENTANMELDUNG

(11) **EP 4 567 022 A1**
(43) Veröffentlichungstag der Anmeldung: **11.06.2025**
(21) Anmeldenummer: 23215244.7
(22) Anmeldetag: 08.12.2023
(51) Int. Cl.: C07C 67/303, C07C 69/75, B01J 23/42, B01J 21/06

(54) **VERFAHREN ZUR STEREOSELEKTIVEN HYDRIERUNG VON PHTHALATEN**

(71) Anmelder: Evonik Oxeno GmbH & Co. KG, 45772 Marl (DE)
(72) Erfinder: QU, Ruiyang, 130024 Changchun, Jilin Province (CN); JENA, Soumyashree, 769001 Sundargarh, Odisha (IN); JACKSTELL, Ralf, 18106 Rostock (DE); JUNGE, Kathrin, 18147 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Die Erfindung betrifft ein Verfahren zur stereoselektiven Hydrierung von Phthalaten, einen Hydrierungskatalysator und ein hydriertes Phthalat.

## Beschreibung

Die vorliegende Erfindung betrifft ein Verfahren zur stereoselektiven Hydrierung von Phthalaten, einen Hydrierungskatalysator und ein hydriertes Phthalat.

Der vorliegenden Erfindung lag die Aufgabe zugrunde ein Verfahren zur stereoselektiven Hydrierung von Phthalaten bereitzustellen.

Diese Aufgabe wird gelöst durch ein Verfahren gemäß Anspruch 1.

Verfahren umfassend die Verfahrensschritte:
a) Vorlegen eines Phthalats gemäß der Formel (I): 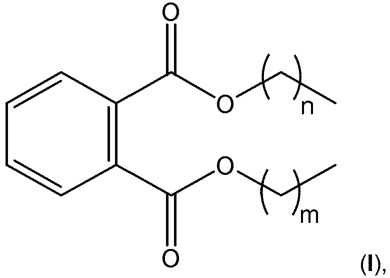 wobei n und m, jeweils unabhängig voneinander, für eine ganze Zahlen von 0 bis 12 stehen;
b) Zugabe von Pt/TiO₂;
c) Zuführen von H₂;
d) Umsetzung des Reaktionsgemisches aus a) bis c), wobei das Phthalat zu einer

Verbindung gemäß Formel (II) umgesetzt wird:

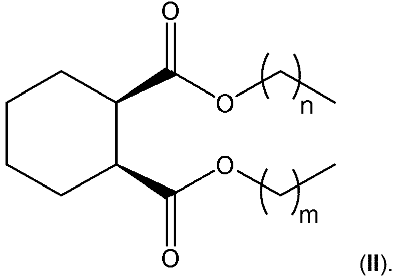

In einer Variante des Verfahrens stehen n und m für die gleiche Zahl.

In einer Variante des Verfahrens stehen n und m für eine Zahl von 4 bis 8.

In einer Variante des Verfahrens weist das Phthalat die Struktur (1) auf:

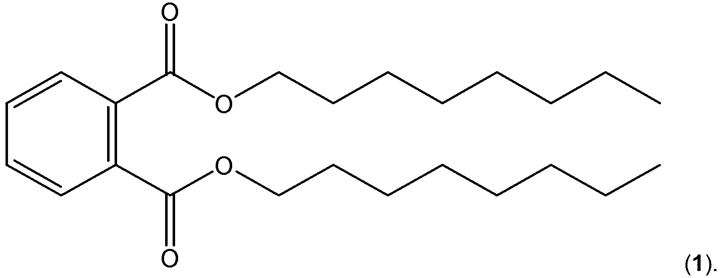

In einer Variante des Verfahrens erfolgt die Zugabe von Pt/TiO₂ in einem Bereich von 0,1 Mol-% Pt bis 5 Mol-% Pt (bezogen auf das Phthalat).

In einer Variante des Verfahrens erfolgt das Zuführen von H₂ mit einem Druck in dem Bereich von 0,5 MPa (5 bar) bis 10 MPa (100 bar).

In einer Variante des Verfahrens erfolgt die Umsetzung des Reaktionsgemisches bei einer Temperatur im Bereich von 20 °C bis 120 °C.

In einer Variante des Verfahrens erfolgt die Umsetzung des Reaktionsgemisches bei einer Temperatur im Bereich von 20 °C bis 100 °C.

In einer Variante des Verfahrens umfasst das Verfahren den zusätzlichen Verfahrensschritt c'):
c') Zugabe eines Lösungsmittels.

In einer Variante des Verfahrens handelt es sich bei dem Lösungsmittel um Cyclohexan.

Neben dem Verfahren wird auch das erhaltene Produkt beansprucht.

Verbindung, welche die Struktur (1) aufweist:

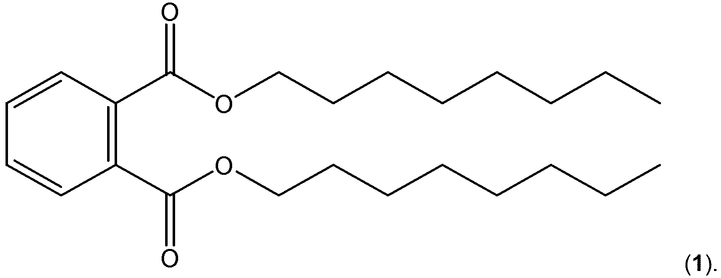

Des Weiteren wird auch die Verwendung des eingesetzten Katalysators in einer entsprechenden Reaktion beansprucht.

Verwendung eines Pt/TiO₂-Katalysators zur Katalyse einer stereoselektiven Hydrierung einer aromatischen Verbindung.

In einer Ausführungsform handelt es ich bei der aromatischen Verbindung um ein Phthalat.

Im Folgenden soll die Erfindung anhand eines Ausführungsbeispiels näher erläutert werden.

### Versuchsbeschreibung

### Herstellung des Katalysators

Es wurde ein geträgerter Pt-Katalysator mit einer Pt-Beladungen von 6 Gew.-% nach der Nassimprägnierungsmethode synthetisiert. Für ein typisches Katalysatorpräparat (0,5 g) wurde die gewünschte Menge an wässriger Wasserstoffhexachlorplatinsäurelösung (7,5 mg Pt-mL-1, Alfa Aesar) mit entionisiertem Wasser auf 16 ml verdünnt und in einem Ölbad unter kräftigem Rühren (800 U/min) 15 Minuten lang auf 60 °C erhitzt, gefolgt von der Zugabe von TiO₂-Träger (P25, Sigma-Aldrich). Die Temperatur wurde 16 Stunden lang bei 60 °C gehalten. Nach dem Abkühlen wurde das Wasser durch Rotationsverdampfung entfernt. Das entstandene Material wurde dann über Nacht bei 110 °C in den Ofen gestellt. Die Probe wurde gründlich gemahlen und dann 4 Stunden lang bei 450 °C mit einer Heizrate von 10 °C-min-1 kalziniert. Schließlich wurde die Probe in einem Durchflussrohrofen unter ständigem Fluss von 5 % H₂/Ar bei 250 °C für 1 h mit einer Heizrate von 5 °C-min-1 reduziert.

### Stereoselektive Hydrierung von Di-n-octylphthalat

### a) Umsetzung mit 0,5 mol-% Pt/TiO₂

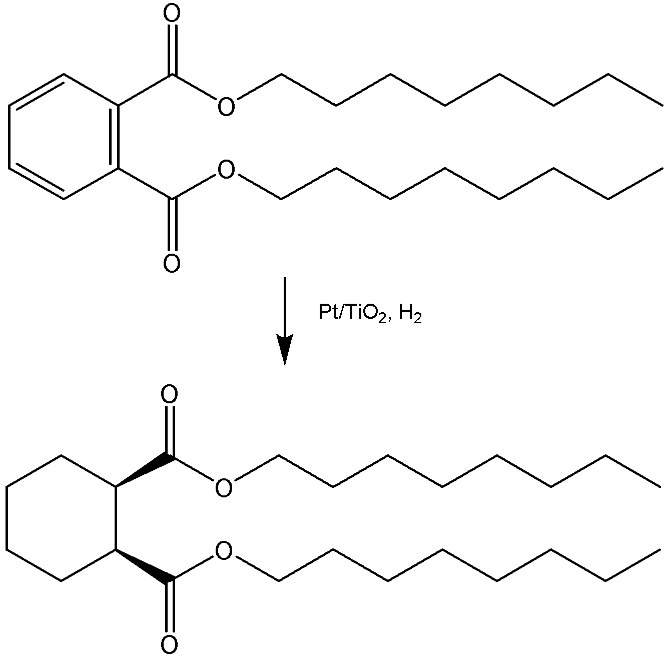

0,5 mol-% Pt/TiO₂ (6 Gew.-%), Substrat (195,3 mg, 0,5 mmol) und Cyclohexan (2 ml) wurden in ein 4-mL-Schraubdeckel-Reaktionsgefäß mit Magnetrührer und Septumkappe gegeben. Dann wurde eine Nadel in das Septum eingeführt, damit H₂ in das Fläschchen eindringen konnte. Das Fläschchen wurden in eine Legierungsplatte eingesetzt und in einen 300-mL-Parr-Autoklaven aus Stahl gestellt. Der Autoklav wurde dreimal mit H₂ bei 10 bar gespült und schließlich auf 10 bar unter Druck gesetzt. Dann wurde dieser in einen Aluminiumblock gestellt und 16 Stunden lang bei 25 °C gehalten. Schließlich wurden die Proben durch Zugabe von Ethylacetat zum Rohgemisch aus dem Autoklaven entfernt und anschließend filtriert, um die festen Katalysatoren mit Hilfe eines Celitkissens abzutrennen. Das Produkt wurde mit Hilfe einer Kieselgelsäulenchromatographie (Ethylacetat/Pentan=1/4) isoliert.

Ausbeute: 96 %.
Optische Reinheit: 99:1

### b) Umsetzung mit 10 g Pt/TiO₂

10 g Pt/TiO₂-Katalysator (6 Gew.-% Pt) und 250 ml des Substrats wurden in einen 450-mL-Edelstahlautoklaven von Parr Instruments gegeben. Der Autoklav wurde dreimal mit 5 bar Wasserstoff gespült. 40 bar Wasserstoff wurden in den Autoklav eingeleitet und die Reaktion lief unter mechanischem Rühren ab. Die Reaktionstemperatur betrug 80 °C und der H₂-Druck lag bei 40 bar. Die Reaktionszeit beträgt 24 h. Nach Beendigung des Gasverbrauchs wurde der Reaktor auf Raumtemperatur abgekühlt und der Druck abgelassen.

Nach der Reaktion wurden der feste Katalysator und das Produkt durch Filtration abgetrennt.

Ausbeute: > 98 %.

### c) Recycling des Katalysators

Der Katalysator wurde mit Cyclohexan gewaschen und anschließend 2 Stunden lang bei 80 °C im Ofen getrocknet. Anschließend wurde der Katalysator gesammelt und gründlich zerkleinert, bevor er über Nacht in eine Vakuumanlage gegeben wurde, um die adsorbierten Spezies vollständig zu entfernen. Der recycelte Katalysator wurde ohne weitere Regenerierung direkt für den nächsten Lauf verwendet. Es wurden vier Zyklen durchgeführt. Bei jedem Durchlauf wurden mehr als 98 % des gewünschten Produkts erhalten.

Wie die Versuchsergebnisse zeigen, wird die Aufgabe durch das erfindungsgemäße Verfahren gelöst.

## Patentansprüche

**1.** Verfahren umfassend die Verfahrensschritte:
a) Vorlegen eines Phthalats gemäß der Formel (I): 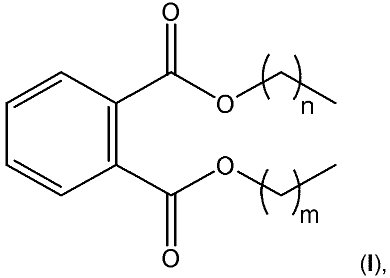 wobei n und m, jeweils unabhängig voneinander, für eine ganze Zahlen von 0 bis 12 stehen;
b) Zugabe von Pt/TiO₂;
c) Zuführen von H₂;
d) Umsetzung des Reaktionsgemisches aus a) bis c), wobei das Phthalat zu einer Verbindung gemäß Formel (II) umgesetzt wird:

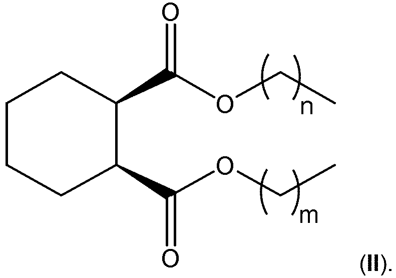

**2.** Verfahren nach Anspruch 1,
wobei n und m für die gleiche Zahl stehen.

**3.** Verfahren nach einem der Ansprüche 1 oder 2,
wobei n und m für eine Zahl von 4 bis 8 stehen.

**4.** Verfahren nach einem der Ansprüche 1 bis 3, wobei das Phthalat die Struktur (1) aufweist:

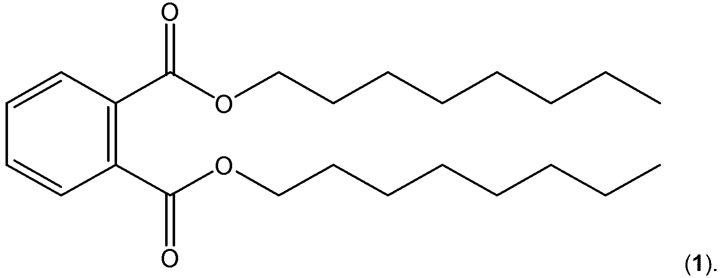

**5.** Verfahren nach einem der Ansprüche 1 bis 4,
wobei die Zugabe von Pt/TiO₂ in einem Bereich von 0,1 Mol-% Pt bis 5 Mol-% Pt (bezogen auf das Phthalat) erfolgt.

**6.** Verfahren nach einem der Ansprüche 1 bis 5,
wobei das Zuführen von H₂ mit einem Druck in dem Bereich von 0,5 MPa (5 bar) bis 10 MPa (100 bar) erfolgt.

**7.** Verfahren nach einem der Ansprüche 1 bis 6,
wobei die Umsetzung des Reaktionsgemisches bei einer Temperatur im Bereich von 20 °C bis 120 °C erfolgt.

**8.** Verfahren nach einem der Ansprüche 1 bis 7,
umfassend den zusätzlichen Verfahrensschritt c'):
c') Zugabe eines Lösungsmittels.

**9.** Verfahren nach Anspruch 8,
wobei es sich bei dem Lösungsmittel um Cyclohexan handelt.

**10.** Verbindung, welche die Struktur (1) aufweist:

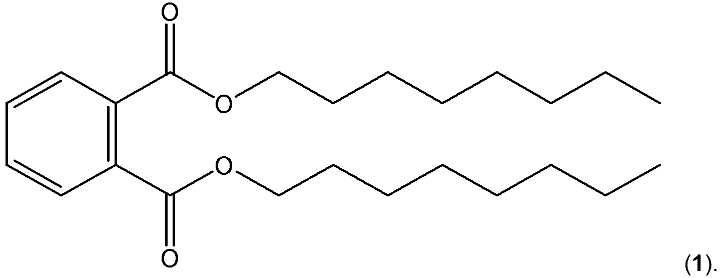

**11.** Verwendung eines Pt/TiO₂-Katalysators zur Katalyse einer stereoselektiven Hydrierung einer aromatischen Verbindung.

**12.** Verwendung nach Anspruch 11,
wobei es ich bei der aromatischen Verbindung um ein Phthalat handelt.

## Geänderte Patentansprüche

### Geänderte Patentansprüche gemäss Regel 137(2) EPÜ.

1. Verfahren umfassend die Verfahrensschritte:
a) Vorlegen eines Phthalats gemäß der Formel (I): 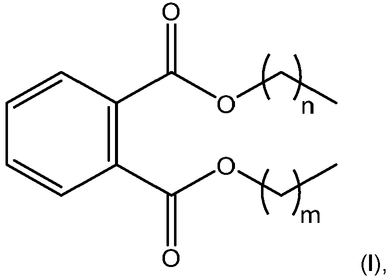 wobei n und m, jeweils unabhängig voneinander, für eine ganze Zahlen von 4 bis 8 stehen;
b) Zugabe von Pt/TiO₂;
c) Zuführen von H₂;
d) Umsetzung des Reaktionsgemisches aus a) bis c), wobei das Phthalat zu einer Verbindung gemäß Formel (II) umgesetzt wird:

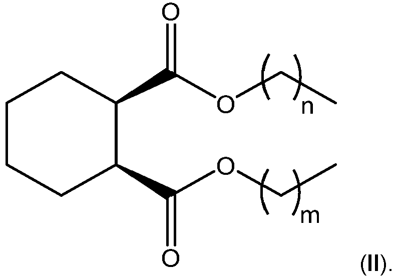

2. Verfahren nach Anspruch 1,
wobei n und m für die gleiche Zahl stehen.

3. Verfahren nach einem der Ansprüche 1 oder 2,
wobei das Phthalat die Struktur (1) aufweist:

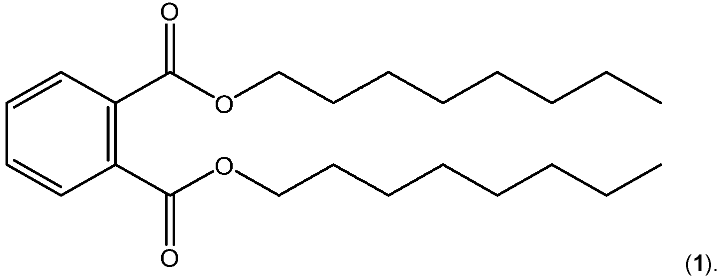

4. Verfahren nach einem der Ansprüche 1 bis 3,
wobei die Zugabe von Pt/TiO₂ in einem Bereich von 0,1 Mol-% Pt bis 5 Mol-% Pt (bezogen auf das Phthalat) erfolgt.

5. Verfahren nach einem der Ansprüche 1 bis 4,
wobei das Zuführen von H₂ mit einem Druck in dem Bereich von 0,5 MPa (5 bar) bis 10 MPa (100 bar) erfolgt.

6. Verfahren nach einem der Ansprüche 1 bis 5,
wobei die Umsetzung des Reaktionsgemisches bei einer Temperatur im Bereich von 20 °C bis 120 °C erfolgt.

7. Verfahren nach einem der Ansprüche 1 bis 6,
umfassend den zusätzlichen Verfahrensschritt c'):
c') Zugabe eines Lösungsmittels.

8. Verfahren nach Anspruch 7,
wobei es sich bei dem Lösungsmittel um Cyclohexan handelt.
